# EUROPÄISCHE PATENTSCHRIFT

(11) **EP 2 455 018 B1**
(45) Veröffentlichungstag und Bekanntmachung des Hinweises auf die Patenterteilung: **21.05.2014**
(21) Anmeldenummer: 10014823.8
(22) Anmeldetag: 22.11.2010
(51) Int. Cl.: A61B 17/34, A61B 17/00

(54) **Trokarhülse zum Einsetzen in eine Hautinzision**
Trocar sheath for insertion into a skin incision
Gaine de trocart pour l'introduction dans une incision de peau

(43) Veröffentlichungstag der Anmeldung: 23.05.2012
(73) Patentinhaber: Karl Storz GmbH & Co. KG, 78532 Tuttlingen (DE)
(72) Erfinder: Oberländer, Martin, 78532 Tuttlingen (DE); Portscheller, Christian, 78532 Tuttlingen (DE); Sauer, Michael, 78532 Tuttlingen (DE); Wagner, Sebastian, 78532 Tuttlingen (DE)

(56) Entgegenhaltungen:
- NL-A- 9 302 140
- US-A- 5 176 649
- US-A- 6 004 303
- US-A1- 2003 216 770
- US-A1- 2003 233 115
- US-A1- 2005 192 483
- US-A1- 2006 036 277

## Beschreibung

Die Erfindung betrifft eine Trokarhülse zum Einsetzen in eine Hautinzision, mit einem hohlen Schaft, über den mindestens ein medizinisches Instrument in ein Untersuchungsgebiet einführbar ist.

Bei der endoskopischen Chirurgie, insbesondere der Laparoskopie, ist es üblich, zunächst eine Hautinzision auszubilden, über die nachfolgend die chirurgischen Instrumente in das Untersuchungsgebiet eingeführt werden. Zum Aufweiten der Hautinzision wird dazu eine einen hohlen Schaft aufweisende Trokarhülse in die Hautinzision eingesetzt, über die nachfolgend die medizinischen Instrumente in das Untersuchungsgebiet eingeführt werden. Um das Untersuchungsgebiet, beispielsweise den Bauchraum, für die Untersuchung und/oder die Operation zu erweitern, wird zur Ausbildung eines Pneumoperitoneums ein Fluid in den Bauchraum geleitet und dieser aufgebläht. Da das solchermaßen in den Bauchraum eingeleitete Fluid durch die in die Hautinzision eingeführte Trokarhülse sofort wieder entweichen würde, sind im Inneren der Trokarhülsen Ventilmittel angeordnet, die das freie Lumen des Schaftes der Trokarhülsen fluiddicht abdichten, jedoch gleichzeitig das Einführen der medizinischen Instrumente erlauben.

Diese Trokarhülsen haben sich in der Praxis durchaus bewährt, jedoch stellt die Ausbildung der Ventilmittel in den Trokarhülsen immer einen fertigungstechnischen Aufwand dar, der auch mit einem gewissen Kostenaufwand verbunden ist.

US-A-5176649 offenbart eine Trokarhülse gemäß dem Oberbegriff des Anspruchs 1.

Davon ausgehend liegt der Erfindung die **Aufgabe** zugrunde, eine Trokarhülse der eingangs genannten Art zu schaffen, die keiner Ventileinrichtungen bedarf.

Die **Lösung** dieser Aufgabenstellung ist dadurch gekennzeichnet, dass zumindest der Schaft aus einem elastisch verformbaren Material besteht, wobei die Elastizität des Schaftmaterials so bemessen ist, dass der hohle Schaft ohne eingesetztes medizinisches Instrument durch die Gewebespannung des den Schaft umgebenden Gewebes der Hautinzision fluiddicht zusammendrückbar ist und bei eingeführtem medizinischen Instrument so zusammendrükbar ist, dass das Schaftmaterial formschlüssig und fluiddicht an der Außenkontur des medizinischen Instruments anliegt.

Durch die Ausbildung zumindest des Schaftes aus einem elastisch verformbaren Material, das sich durch die Gewebespannung des den Schaft umgebenden Gewebes der Hautinzision selbsttätig fluiddicht verschließt ist es erstmalig möglich, eine Trokarhülse ganz ohne eingebaute Ventilmittel auszubilden.

Gemäß einer praktischen Ausführungsform der Erfindung wird vorgeschlagen, dass zumindest der in der Hautinzision anzuordnende hohle Schaft der Trokarhülse aus einem Kunststoffmaterial, insbesondere PE, PU, Silikon, TPE oder einem thermoplastischen Elastomer besteht. Gemäß dieser Ausführungsform ist es möglich, den Schaft beispielsweise als extrudierten Schlauch auszubilden.

Mit einer alternativen Ausführungsform der Erfindung wird vorgeschlagen, dass zumindest der in der Hautinzision anzuordnende hohle Schaft aus einem federelastischen Metalldraht, insbesondere NiTinol besteht.

Um den elastisch verformbaren Schaft der Trokarhülse in die Hautinzision einführen zu können, wird mit der Erfindung vorgeschlagen, dass der Schaft mittels mindestens einer Einführhilfe in die Hautinzision einsetzbar ist. Da die Hautinzision ohne eingesetzte Trokarhülse aufgrund der Gewebespannung verschlossen ist, erleichtert die Einführhilfe das Einführen des elastischen Schaftes.

Gemäß einer ersten Ausführungsform der Erfindung wird vorgeschlagen, dass die Einführhilfe als an der Trokarhülse festlegbarer Stab ausgebildet ist, wobei vorteilhafterweise im Stab eine Längsnut zum klemmenden Festlegen der Trokarhülse ausgebildet ist. Über den als Vollmaterial ausgebildeten Stab lässt sich die Gewebeinzision aufweiten und der elastisch verformbare Schaft in die Hautinzision einführen.

Mit einer zweiten Ausführungsform der Erfindung wird vorgeschlagen, dass die Einführhilfe als mindestens ein einstückig mit der Trokarhülse verbundener Stab ausgebildet ist.

Bei der Verwendung einer als Stab ausgebildeten Einfiihrhilfe wird mit der Erfindung weiterhin vorgeschlagen, dass zumindest der Schaft zum Einführen in die Hautinzision um den Stab aufwickelbar ist. Im aufgewickelten Zustand wird der Stab in die Hautinzision eingesteckt und nachfolgend der elastisch verformbare Schaft der Trokarhülse so abgewickelt, dass dieser bereits zum Einführen der medizinischen Instrumente in der Hautinzision angeordnet ist.

Schließlich wird mit der Erfindung vorgeschlagen, dass das mindestens eine in die Trokarhülse einführbare medizinische Instrument über ein Spreizwerkzeug, beispielsweise einen Trichter, in die Trokarhülse einführbar ist. Durch das in das proximale Ende des Schaftes einsetzbare Spreizwerkzeug wird das freie Lumen des Schaftes so aufgeweitet, dass das mindestens eine medizinische Instrument in den Schaft der Trokarhülse eingeschoben werden kann, wobei das Lumen des Schaftes im Bereich der Hautinzision entweder durch das Spreizwerkzeug oder durch das eingeführte medizinische Instrument aufgeweitet wird.

Weitere Merkmale und Vorteile der Erfindung ergeben sich anhand der zugehörigen Zeichnungen, in denen drei Ausführungsbeispiele einer erfindungsgemäßen Trokarhülse nur beispielhaft dargestellt ist, ohne die Erfindung auf dieses Ausführungsbeispiel zu beschränken. In den Zeichnungen zeigt:
- Fig. 1: eine Schnittdarstellung, eine erfindungsgemäße Trokarhülse im in eine Hautinzision in der Bauchdecke eingesetzten Zustand darstellend;
- Fig. 2a: eine Draufsicht auf eine erste Ausführungsform einer erfindungsgemäßen Trokarhülse im geschlossenen Zustand;
- Fig. 2b: eine Ansicht gemäß Fig. 2a, jedoch die Trokarhülse im geöffneten Zustand darstellend;
- Fig. 3a: eine Ansicht gemäß Fig. 2a, die Trokarhülse mit angefügter Einführhilfe darstellend;
- Fig. 3b: eine Ansicht gemäß Fig. 3a, jedoch die mit der Einführhilfe versehene Trokarhülse im geöffneten Zustand darstellend;
- Fig. 3c: eine Draufsicht auf eine Trokarhülse gemäß Fig. 3a, jedoch die auf die Einführhilfe aufgewickelte Einführstellung darstellend;
- Fig. 4a: eine Draufsicht auf eine zweite Ausführungsform einer erfindungsgemäßen Trokarhülse im geschlossenen Zustand;
- Fig. 4b: eine Ansicht gemäß Fig. 4a, jedoch die Trokarhülse im geöffneten Zustand darstellend;
- Fig. 5a: eine Draufsicht auf eine dritte Ausführungsform einer erfindungsgemäßen Trokarhülse im geschlossenen Zustand und
- Fig. 5b: eine Ansicht gemäß Fig. 5a, jedoch die Trokarhülse im geöffneten Zustand darstellend.

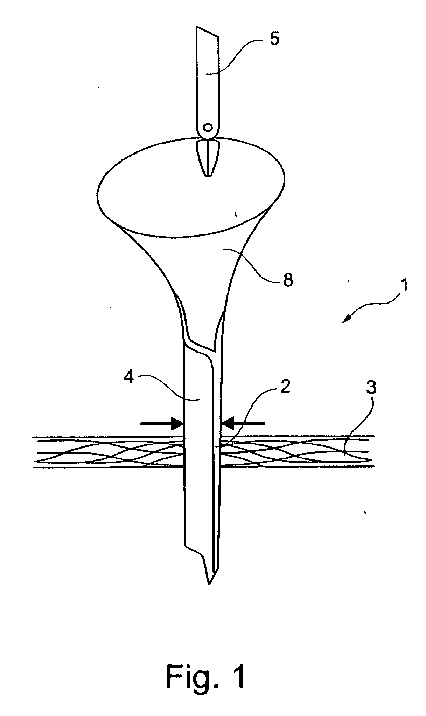

In den Abbildungen Fig. 1 bis Fig. 5b sind beispielhaft schematisch drei Ausführungsformen von Trokarhülsen 1 dargestellt, die für die Verwendung bei endoskopischen Operationen ausgebildet sind. Zu Beginn der Operation wird beispielsweise mittels eines Trokardoms eine Hautinzision 2 in der Bauchdecke 3 eines Patienten ausgebildet. In diese Hautinzision 2 wird nachfolgend die Trokarhülse 1 so eingesetzt, dass ein Schaft 4 der Trokarhülse 1 die Hautinzision 2 passiert, um über die Trokarhülse 1 medizinische Instrumente 5 in das Operationsgebiet einzuführen.

Um das Untersuchungsgebiet, beispielsweise den Bauchraum, für die Untersuchung und/oder die Operation zu erweitern, wird zur Ausbildung eines Pneumoperitoneums ein Fluid in den Bauchraum geleitet und dieser aufgebläht. Da das solchermaßen in den Bauchraum eingeleitete Fluid durch die in die Hautinzision 2 eingeführte Trokarhülse 1 sofort wieder entweichen würde, ist es erforderlich, die Trokarhülsen 1 durch geeignete Mittel fluiddicht abdichtend auszubilden.

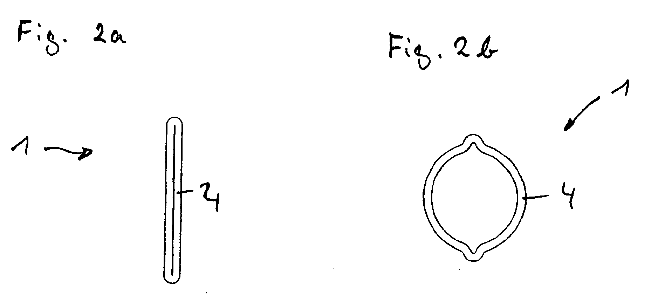

Bei den in Abbildungen dargestellten Trokarhülsen 1 bestehen zumindest die Schäfte 4 der Trokarhülsen 1 aus einem elastisch verformbaren Material, dessen Elastizität so bemessen ist, dass der hohle Schaft 4 ohne in den Schaft 4 eingesetztes medizinisches Instrument 5 durch die Gewebespannung des den Schaft 4 umgebenden Gewebes der Hautinzision 2 fluiddicht zusammengedrückt wird, wie dies den Abbildungen Fig. 2a, 3a, 4a und 5a zu entnehmen ist.

Durch diese Ausbildung der Schäfte 4 der Trokarhülsen 1 aus einem elastisch verformbaren Material, ist es möglich, Trokarhülsen 1 ganz ohne eingebaute Ventilmittel auszubilden und trotzdem das Pneunoperitoneum aufrecht zu erhalten.

Bei in die Trokarhülse 1 eingeführtem medizinischen Instrument 5 wird der hohle Schaft 4 durch die Gewebespannung des den Schaft 4 umgebenden Gewebes der Hautinzision 2 so zusammengedrückt, dass das Schaftmaterial formschlüssig und fluiddicht an der Außenkontur des medizinischen Instruments 5 anliegt.

Im Falle der in den Abbildungen dargestellten Trokarhülsen 1 bestehen die gesamten Trokarhülsen 1 aus elastisch verformbaren Material. Wie aus Fig. 2a bis 5b ersichtlich, sind bei den dargestellten Ausführungsformen die Trokarhülsen 1 als Schläuche aus einem Kunststoffmaterial, aus einem Kunststoffmaterial, insbesondere PE, PU, Silikon, TPE oder einem thermoplastischen Elastomer, ausgebildet. Alternativ zur Ausbildung der gesamten Trokarhülse 1 oder zumindest des Schaftes 4 als Schlauch ist es jedoch auch möglich, die Trokarhülse 1 so auszubilden, dass zumindest der hohle Schaft 4 aus einem federelastischen Metalldraht, insbesondere NiTinol, besteht.

Gemäß einer weiteren, nicht dargestellten Ausführungsform besteht nur der die Hautinzision 2 passierende Schaft 4 der Trokarhülse 1 aus einem elastisch verformbaren Material, das sich durch die Gewebespannung des den Schaft 4 umgebenden Gewebes der Hautinzision 2 fluiddicht zusammendrücken lässt. Das sich an den Schaft 4 anschließende proximale und/oder das distale Ende der Trokarhülse 1 besteht bei dieser Ausführungsform aus einem anderen, in der Regel steiferen Material, um beispielsweise das Einführen der Trokarhülse 1 in die Hautinzision 2 und/oder das Einführen eines medizinischen Instruments 5 in die Trokarhülse 1 zu erleichtern.

In den Abbildungen Fig. 2b, 3b, 4b und 5b, die jeweils die Trokarhülsen 1 bzw. Schäfte 4 im geöffneten Zustand zeigen, wurde aus Gründen der besseren Übersichtlichkeit, sowie zum Verdeutlichen der elastischen Verformbarkeit der Trokarhülsen 1 bzw. Schäfte 4 auf die Darstellung der medizinischen Instrumente 5 verzichtet, durch welche das elastisch verformbare Material der Trokarhülsen 1 bzw. Schäfte 4, gegen die Rückstellkraft der Gewebespannung des die Schäfte 4 umgebenden Gewebes der Hautinzision 2 von der in den Abbildungen Fig. 2a, 3a, 4a und 5a dargestellten geschlossenen Position in die geöffnete Position überführt werden.

Um das Einführen der Trokarhülsen 1 in die Hautinzision 2 zu erleichtern, insbesondere dann, wenn nicht nur der Schaft 4 der Trokarhülse 1, sondern die gesamte Trokarhülse 1 aus dem elastisch verformbaren Material besteht, ist eine Einführhilfe 6 vorgesehen, die bei den dargestellten Ausführungsformen als Stab 6 ausgebildet ist. Da die Hautinzision 2 ohne eingesetzte Trokarhülse 1 aufgrund der Gewebespannung verschlossen ist, erleichtert die Einführhilfe 6 das Einführen des elastischen Schaftes 4 der Trokarhülse 1 in die Hautinzision.

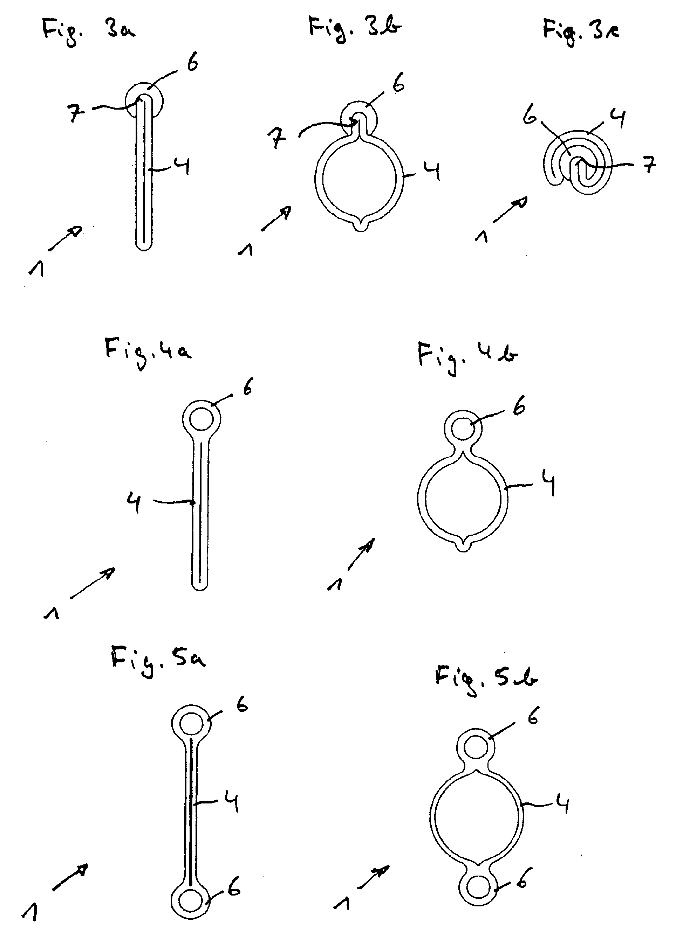

Gemäß der in den Abbildungen Fig. 3a-3c dargestellten ersten Ausführungsform ist die Einführhilfe 6 als an der Trokarhülse 1 festlegbarer Stab 6 ausgebildet, wobei im Stab 6 eine Längsnut 7 zum klemmenden Festlegen der Trokarhülse 1 ausgebildet ist.

Bei beiden in den Abbildungen Fig. 4a-5b dargestellten Ausführungsformen ist die Einführhilfe 6 als einstückig mit der Trokarhülse 1 verbundener Stab 6 ausgebildet, die beispielsweise mittels Extrudieren einstückig mit dem als Schlauch ausgebildeten Schaft 4 der Trokarhülse 1 herstellbar ist. Die beiden dargestellten Ausführungsformen unterscheiden sich dadurch voneinander, dass die in den Abbildungen Fig. 4a und 4b dargestellte Trokarhülse 1 nur einseitig eine angeformte Einführhilfe 6 aufweist, wohingegen bei der in Fig. 5a und 5b dargestellten Ausführungsform der schlauchförmige Schaft 4 der Trokarhülse 1 zwischen zwei angeformten Einführhilfen 6 aufgespannt ist.

Um das Einführen des mindestens einen medizinischen Instruments 5 in die Trokarhülsen 1 zu erleichtern, insbesondere dann, wenn nicht nur der Schaft 4 der Trokarhülse 1, sondern die gesamte Trokarhülse 1 aus dem elastisch verformbaren Material besteht, kann ein Spreizwerkzeug 8 verwendet werden, das bei de in Fig. 1 dargestellten Ausführungsform als Trichter 8 ausgebildet ist.

Bei der Verwendung der als Stab 6 ausgebildeten Einführhilfe 6 erfolgt das Einsetzen der Trokarhülse 1 in die Hautinzision 2 derart, dass das elastisch verfombare Material des Schaftes 4 um den Stab 6 aufgewickelt wird, wie dies in Abbildung Fig. 3c dargestellt ist. Im aufgewickelten Zustand wird der Stab 6 in die Hautinzision 2 eingesteckt und nachfolgend der elastisch verformbare Schaft 4 der Trokarhülse 1 so abgewickelt, dass dieser bereit zum Einführen der medizinischen Instrumente 5 in der Hautinzision 2 angeordnet ist.

Eine wie voranstehend beschrieben ausgebildete Trokarhülse 1 zeichnet sich dadurch aus, dass die erforderliche Fluiddichtigkeit auch ohne den Einbau von Ventilmitteln erzielbar ist.

### Bezugszeichenliste

- 1: Trokarhülse
- 2: Hautinzision
- 3: Bauchdecke
- 4: Schaft
- 5: medizinisches Instrument
- 6: Einführhilfe / Stab
- 7: Längsnut
- 8: Spreizwerkzeug / Trichter

## Patentansprüche

1. Trokarhülse zum Einsetzen in eine Hautinzision (2), mit einem hohlen Schaft (4),
über den mindestens ein medizinisches Instrument (5) in ein Untersuchungsgebiet einführbar ist,
**dadurch gekennzeichnet,**
**dass** zumindest der Schaft (4) aus einem elastisch verformbaren Material besteht, wobei die Elastizität des Schaftmaterials so bemessen ist, dass das den Schaft (4) umgebende Gewebe der Hautinzision (2) den hohlen Schaft (4) ohne eingesetztes medizinisches Instrument (5) durch die Gewebespannung fluiddicht zusammendrückt und bei eingeführtem medizinischen Instrument (5) so zusammendrückt, dass das Schaftmaterial formschlüssig und fluiddicht an der Außenkontur des medizinischen Instruments (5) anliegt.

2. Trokarhülse nach Anspruch 1, **dadurch gekennzeichnet, dass** zumindest der hohle Schaft (4) aus einem Kunststoffmaterial, insbesondere PE, PU, Silikon, TPE oder einem thermoplastischen Elastomer, besteht.

3. Trokarhülse nach Anspruch 1, **dadurch gekennzeichnet, dass** zumindest der hohle Schaft (4) aus einem federelastischen Metalldraht, insbesondere NiTinol, besteht.

4. Trokarhülse nach einem der Ansprüche 1 bis 3, **dadurch gekennzeichnet, dass** die Trokarhülse (1) mittels mindestens einer Einführhilfe (6) in die Hautinzision (2) einsetzbar ist.

5. Trokarhülse nach Anspruch 4, **dadurch gekennzeichnet, dass** die Einführhilfe (6) als an der Trokarhülse (1) festlegbarer Stab (6) ausgebildet ist.

6. Trokarhülse nach Anspruch 5, **dadurch gekennzeichnet, dass** im Stab (6) eine Längsnut (7) zum klemmenden Festlegen der Trokarhülse (1) ausgebildet ist.

7. Trokarhülse nach Anspruch 4, **dadurch gekennzeichnet, dass** die Einführhilfe (6) als mindestens ein einstückig mit der Trokarhülse (1) verbundener Stab (6) ausgebildet ist.

8. Trokarhülse nach einem der Ansprüche 5 bis 7, **dadurch gekennzeichnet, dass** zumindest der Schaft (4) zum Einführen in die Hautinzision (2) um den Stab (6) wickelbar ist.

9. Trokarhülse nach einem der Ansprüche 1 bis 8, **dadurch gekennzeichnet, dass** das mindestens eine in die Trokarhülse (1) einführbare medizinische Instrument (5) über ein Spreizwerkzeug (8), beispielsweise einen Trichter (8), in die Trokarhülse (1) einführbar ist.

## Claims

1. Trocar sheath for insertion into a skin incision (2), with a hollow shaft (4) via which at least one medical instrument (5) can be inserted into an examination site, **characterized in that** at least the shaft (4) is made of an elastically deformable material, the elasticity of the shaft material being such that, without an inserted medical instrument (5), the tissue of the skin incision (2) surrounding the shaft (4) presses the hollow shaft (4) together in a fluid-tight manner and, when a medical instrument (5) is inserted, presses together such that the shaft material bears with a form fit and in a fluid-tight manner on the outer contour of the medical instrument (5).

2. Trocar sheath according to Claim 1, **characterized in that** at least the hollow shaft (4) is made of a plastics material, in particular PE, PU, silicone, TPE or a thermoplastic elastomer.

3. Trocar sheath according to Claim 1, **characterized in that** at least the hollow shaft (4) is made of a resilient metal wire, in particular Nitinol.

4. Trocar sheath according to one of Claims 1 to 3, **characterized in that** the trocar sheath (1) can be fitted into the skin incision (2) by means of at least one insertion aid (6).

5. Trocar sheath according to Claim 4, **characterized in that** the insertion aid (6) is designed as a rod (6) that can be fixed on the trocar sheath (1).

6. Trocar sheath according to Claim 5, **characterized in that** a longitudinal groove (7) is formed in the rod (6) for securely clamping the trocar sheath (1).

7. Trocar sheath according to Claim 4, **characterized in that** the insertion aid (6) is designed as at least one rod (6) connected integrally to the trocar sheath (1).

8. Trocar sheath according to one of Claims 5 to 7, **characterized in that** at least the shaft (4) can be wound around the rod (6) for insertion into the skin incision (2).

9. Trocar sheath according to one of Claims 1 to 8, **characterized in that** the at least one medical instrument (5) for insertion into the trocar sheath (1) can be inserted into the trocar sheath (1) via an expansion tool (8), for example a funnel (8).

## Revendications

1. Gaine de trocart pour l'insertion dans une incision de peau (2), comprenant une tige creuse (4), par le biais de laquelle au moins un instrument médical (5) peut être introduit dans une zone à examiner,
**caractérisée en ce**
**qu'**au moins la tige (4) se compose d'un matériau déformable élastiquement, l'élasticité du matériau de la tige étant conçue de telle sorte que le tissu de l'incision de peau (2) entourant la tige (4) comprime de manière étanche aux fluides la tige creuse (4) par la tension du tissu lorsque l'instrument médical (5) n'est pas inséré et, lorsque l'instrument médical (5) est inséré, la comprime de telle sorte que le matériau de la tige s'applique par engagement par correspondance de forme de manière étanche aux fluides contre le contour extérieur de l'instrument médical (5).

2. Gaine de trocart selon la revendication 1, **caractérisée en ce qu'**au moins la tige creuse (4) se compose d'un matériau en plastique, en particulier de PE, de PU, de silicone, de TPE ou d'un élastomère thermoplastique.

3. Gaine de trocart selon la revendication 1, **caractérisée en ce qu'**au moins la tige creuse (4) se compose d'un fil métallique élastique à ressort, en particulier de NiTinol.

4. Gaine de trocart selon l'une quelconque des revendications 1 à 3, **caractérisée en ce que** la gaine de trocart (1) peut être insérée dans l'incision de peau (2) au moyen d'au moins un auxiliaire d'introduction (6).

5. Gaine de trocart selon la revendication 4, **caractérisée en ce que** l'auxiliaire d'introduction (6) est réalisé sous forme de barre (6) pouvant être fixée à la gaine de trocart (1).

6. Gaine de trocart selon la revendication 5, **caractérisée en ce qu'**une rainure longitudinale (7) est réalisée dans la barre (6) pour la fixation par serrage de la gaine de trocart (1).

7. Gaine de trocart selon la revendication 4, **caractérisée en ce que** l'auxiliaire d'introduction (6) est réalisé sous forme d'au moins une barre (6) connectée d'une seule pièce à la gaine de trocart (1).

8. Gaine de trocart selon l'une quelconque des revendications 5 à 7, **caractérisée en ce qu'**au moins la tige (4) peut être enroulée autour de la barre (6) pour l'introduction dans l'incision de peau (2).

9. Gaine de trocart selon l'une quelconque des revendications 1 à 8, **caractérisée en ce que** l'au moins un instrument médical (5) pouvant être introduit dans la gaine de trocart (1) peut être introduit dans la gaine de trocart (1) par le biais d'un outil d'écartement (8), par exemple un entonnoir (8).
